# FASCICULE DE BREVET EUROPEEN

(11) **EP 3 280 693 B1**
(45) Date de publication et mention de la délivrance du brevet: **27.11.2019**
(21) Numéro de dépôt: 16729954.4
(22) Date de dépôt: 05.04.2016
(51) Int. Cl.: C07C 51/34, C07C 55/18, C07C 53/126

(54) **MÉTHODE DE SYNTHÈSE SIMULTANÉE DE L'ACIDE AZELAÏQUE ET DE L'ACIDE PELARGONIQUE PAR L'OZONE**
OZONBASIERTES VERFAHREN ZUR SIMULTANEN SYNTHESE VON AZELAINSÄURE UND PELARGONSÄURE
OZONE-BASED METHOD FOR THE SIMULTANEOUS SYNTHESIS OF AZELAIC ACID AND PELARGONIC ACID

(30) Priorité: 07.04.2015 FR 1552960
(43) Date de publication de la demande: 14.02.2018
(73) Titulaire: Institut Polytechnique Unilasalle, 60026 Beauvais Cedex (FR)
(72) Inventeur: BONHOURE, Jean-Paul, 95390 Saint-Prix (FR); HOANG, Levinh, 60000 Beauvais (FR); COSTE, Christian, Ulysse, 84870 Loriol du Comtat (FR); AUSSENAC, Thierry, Robert, 60000 Beauvais (FR)
(74) Mandataire: Hart-Davis, Jason
(86) Numéro de dépôt international: PCT/FR2016/050775
(87) Numéro de publication internationale: WO 2016/162628

(56) Documents cités:
- DE-C- 565 158
- GB-A- 810 571
- GB-A- 841 653
- US-A1- 2012 245 375

## Description

### Domaine de l'invention

La présente invention se rapporte à l'utilisation de l'ozone pour la synthèse simultanée de l'acide azélaïque et de l'acide pélargonique à partir de l'acide oléique. Elle concerne en particulier un procédé de synthèse mettant en œuvre l'ozone qui permet d'atteindre un rendement équimolaire élevé en acide azélaïque (obtenue sous forme cristalline) et en acide pélargonique (obtenu sous forme liquide) sans employer de solvant organique ni catalyseur, ni acide fort ni base forte et avec une consommation d'énergie modérée.

### Arrière-plan téchnologique

Les acides azélaïque (C₉H₁₆O₄; N°CAS 123-99-9) (a) et pélargonique (C₉H₁₈O₂; N°CAS 112-05-0) (b) présentent les structures suivantes :

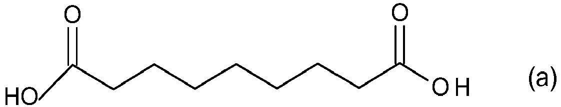

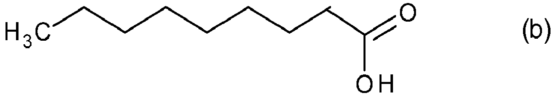

Un mélange de ces acides peut être obtenu par coupure oxydante de la double liaison d'une chaîne insaturée d'un acide gras d'origine naturelle, notamment l'acide oléique. Les diacides organiques présentent un grand intérêt, pouvant trouver usage notamment: (i) en tant qu'agents réticulants, (ii) dans la synthèse de polymères.

La variété des réactifs utilisables pour la coupure oxydante d'une double liaison C=C est très importante : permanganate de potassium, périodate de sodium (associé le plus souvent au chlorure de ruthénium), peroxyde d'hydrogène, acide péracétique, hypochlorite de sodium. Toutefois, le plus souvent, ces synthèses n'ont de réelles applications qu'à l'échelle du laboratoire (de l'ordre du milligramme ou du gramme mis en jeu), car les réactifs impliqués, souvent assistés de catalyseurs coûteux, ne seraient pas d'un emploi réaliste à l'échelle industrielle.

L'ozone est apparu très tôt comme un réactif performant pour cette réaction de coupure oxydante. Les difficultés essentielles liées à son usage résident dans la maîtrise de la conduite de la réaction et la définition d'un milieu réactionnel approprié.

Le brevet US 2 813 113 décrit la coupure oxydante par l'ozone de l'acide oléique afin de synthétiser l'acide azélaïque. L'acide pélargonique, issu du clivage oxydant de l'acide oléïque, peut être recyclé et utilisé comme solvant, notamment pour réduire la viscosité des ozonides issus de la première phase de la réaction de l'acide oléique avec l'ozone.

Les acides gras saturés ne réagissent pas avec l'ozone, et leurs caractéristiques de volatilité et d'inflammabilité permettent de garantir un procédé convenablement sûr. La réaction d'oxydation complète est en effet fortement exothermique et, pour cette raison, le choix du solvant doit être fait de manière à prévenir tout risque d'explosion ou de production de vapeurs. En effet, l'obstacle principal lié à l'usage de l'ozone comme oxydant des acides gras et lipides insaturés est lié à sa grande réactivité. L'usage d'un solvant adéquat s'impose donc généralement pour permettre de limiter l'exothermicité de la réaction d'oxydation. Mal maîtrisée, l'exothermicité induite représente un risque important et, au mieux, génère des pertes de rendement élevées.

Le brevet US 4 287 130 décrit l'usage d'acides gras comme solvants pour l'ozonolyse d'oléfines. Les brevets US 7 825 277 et US 5 278 327 décrivent également l'ozonolyse de l'acide oléique en présence d'acide pélargonique.

Les brevets US 8 871 960, US 8 877 952, US 8 624 047 et US 8 859 794 décrivent l'oxydation directe d'huiles, par ozonolyse en présence d'acides de Lewis (tout particulièrement BF₃), suivie d'une décomposition de l'ozonide produit en conditions choisies pour permettre sa fonctionnalisation. La demande de brevet US 2007/0175793 a, globalement, le même objectif, c'est à dire la coupure oxydante de triglycérides sans destruction du motif glycérol, et ce avant fonctionnalisation et polymérisation. Le brevet US 5 399 749 décrit l'ozonolyse de l'acide oléique en présence d'une zéolite catalyseur.

La demande de brevet US 2003/0165685 décrit une synthèse de type « one-pot », consistant en l'ozonolyse d'un acide gras insaturé, puis l'amination du brut réactionnel conduisant à des α-aminoacides.

Les demandes de brevet WO 2012/103310, WO 2012/103325 et WO 2012/103330 décrivent l'ozonolyse d'acides gras tels que l'acide oléique, de manière facultative en présence de l'acide pélargonique en tant que solvant.

La demande de brevet US 2012/0245375 porte sur le traitement par l'ozone de dérivés d'acides gras dans un mélange solvant constitué d'eau et d'un acide gras (préférentiellement à courte chaîne), avec ou sans addition d'acide fort. Les brevets GB 841 653 et DE 565 158 décrivent également le traitement par l'ozone de dérivés d'acides gras dans un mélange solvant constitué d'eau et d'un acide gras, à savoir l'acide caproïque et l'acide acétique, respectivement. Le brevet GB 810 571 décrit une réaction de l'ozone avec un mélange d'acide oléique et d'eau, suivi par un rajout de soude et d'acide chlorhydrique.

### Résumé de l'invention

La présente invention se rapporte à une nouvelle méthode de synthèse simultanée par l'ozone des acides azélaïque et pélargonique. La présente invention se rapporte ainsi à un procédé de préparation simultanée des acides azélaïque et pélargonique tel que défini dans la revendication 1 et comprenant les étapes successives suivantes :
(a) fourniture d'une solution mère liquide d'acide oléique ;
(b) dispersion d'une quantité d'eau dans la solution mère liquide d'acide oléique fournie à l'étape (a) ;
(c) mise en contact de la dispersion d'eau dans la solution mère liquide d'acide oléique, obtenue à l'étape (b), avec de l'ozone gazeux.

Selon un mode de réalisation, le produit de départ pour la synthèse des acides azélaïque et pélargonique est une solution riche (au-delà de 75% m/m, de préférence au moins 80% m/m) d'acide oléique (d'origine végétale diverse).

Selon un procédé préférentiel de l'invention, le produit de départ correspond à une solution d'acide oléique dont 80% par masse est constituée d'acide oléique présent). Un produit de départ pouvant convenir dans ce contexte est la préparation dénommée Oléine V2 fabriqué par l'entreprise Stéarinerie DUBOIS. Dans les préparations industrielles contenant majoritairement de l'acide oléique, les produits secondaires sont généralement essentiellement d'autres acides gras saturés et insaturés. Un produit courant à base d'acide oléique, qui peut avantageusement être utilisé dans la présente invention, comprend (rapports massiques) 80% m/m en acide oléique, 11% m/m en acide linoléique, 9% m/m d'acides gras saturés (acide palmitique, acide stéarique...). Dans la présente invention, ce type de produit de départ, un liquide que l'on peut considérer comme une solution de divers acides gras dans l'acide oléique, est appelée « solution mère liquide d'acide oléique ». Le pourcentage maximal d'eau dans le produit acide oléique de départ, c'est-à-dire dans la solution mère liquide d'acide oléique, avant formation de la dispersion à l'étape (b) du procédé est de préférence inférieur à 10% m/m, plus préférentiellement inférieur à 5% m/m, et encore plus préférentiellement inférieur à 1% m/m. Dans un mode de réalisation avantageux, la solution d'acide oléique est substantiellement anhydre, ne contenant pas d'eau si ce n'est sous forme de traces infimes difficilement quantifiables.

La Demanderesse a découvert que la réaction d'oxydation par l'ozone d'une solution d'acide oléique pouvait être conduite en toute sécurité et avec un excellent rendement en utilisant l'eau comme milieu solvant, dès le début de réaction. Il est en effet avantageux dans le cadre de l'invention d'utiliser l'eau comme unique solvant rajouté à l'acide oléique à transformer, sans rajout de solvant organique quelconque tel que notamment les acides carboxyliques C1 à C10 (acides acétique, propionique, caproïque (hexanoïque), pélargonique (nonanoïque) etc.). A cette fin il est avantageux que la dispersion de l'eau dans l'acide oléique soit maintenue homogène par application d'un fort coefficient de cisaillement correspondant à un ASN (Apport Spécifique Nominal) significatif, de l'ordre de 30 à 80 Watts/litre de dispersion, préférentiellement de 30 à 60 Watts/litre de dispersion, plus préférentiellement de 40 à 60 Watts/litre de dispersion, et dans un mode préférentiel entre 40 et 50 Watts/litre de dispersion. La vitesse de rotation préférentielle des mobiles d'agitation est préférentiellement d'au moins 170 rpm et d'au plus 270 rpm, des vitesses de rotation préférentielles des mobiles d'agitation de l'ordre de 220 rpm étant particulièrement appropriées.

Concernant l'eau ajoutée dans la solution mère liquide d'acide oléique à l'étape (b) du procédé selon la présente invention, l'eau pure (déionisée ou déminéralisée) sera avantageusement utilisée. L'eau potable peut aussi être utilisée. Le pourcentage massique d'eau par rapport à la masse de solution mère liquide d'acide oléique lors de la réalisation de la dispersion à l'étape (b) est d'au moins 15% et d'au plus 34%.

L'introduction d'ozone gazeux dans le milieu réactionnel (dispersion homogène d'eau et d'acide oléique) induit la réaction d'oxydation. L'ozone injecté sous forme gazeux (gaz vecteur + ozone généré), initie la réaction d'oxydation à la condition que les fines bulles d'ozone et de son gaz vecteur intéressent la totalité de la masse d'acide oléique et d'eau (la dispersion). La dispersion des fines bulles d'ozone et de son gaz vecteur est favorisée par une agitation et un cisaillement important qui sont, de manière avantageuse, appliqués à la masse totale de la dispersion par un dispositif de turbine de type Rushton bi ou tri-étagé, accompagné d'une mise en vitesse intermédiaire par un jeu de turbines d'accélération intercalé entre les étages de turbines Rushton. D'autres types de dispositifs peuvent également convenir dans cette fonction, et notamment tout dispositif capable de générer un ASN compris entre 30 et 80 Watts/litre de dispersion, selon un mode préférentiel entre 30 et 60 Watts/litre de dispersion et assurant un fort coefficient de cisaillement (forme des mobiles d'agitation). Un tel dispositif peut être représenté par une turbine FRING'S.

Dans ces conditions, la totalité du volume de la dispersion est parcourue par le flux ascendant de fines bulles, qui assure une excellente surface d'échange et un excellent coefficient global de transfert (*K_{L}. a*), entre les fines bulles de gaz (ozone + gaz vecteur) et le milieu liquide (dispersion acide oléique + eau).

Durant la réaction d'oxydation générée par l'introduction d'ozone gazeux dans le réacteur, il apparait comme produit de la réaction primaire un mélange équimolaire d'acide pélargonique et d'acide azélaïque, sans introduction préalable d'acide pélargonique comme solvant ni d'autres solvants organiques, et sans addition de catalyseurs ni d'acides forts ni de bases fortes.

Le procédé selon l'invention permet en effet de ne pas employer de solvant organique, par exemple les acides carboxyliques (dont l'acide pélargonique) qui sont connus dans des réactions d'ozonation, ni d'autres solvants organiques (alcools, éthers, cétones, esters, hydrocarbures (éventuellement chlorés) aliphatiques ou aromatiques, sulfoxydes, acétonitrile, amides etc.). On peut noter que l'utilisation de solvants organiques, à part le coût et les dangers d'inflammation, soulève des problèmes liés à la contamination. Par exemple, les solvants chlorés présentent le risque de résidus chlorés dans le produit final.

Le procédé selon l'invention de transformation d'acide oléique en acides azélaïque et pélargonique est mené jusqu'à l'obtention des produits finals (acides azélaïque et pélargonique) en utilisant l'eau comme unique solvant rajouté au mélange réactionnel, sans rajout à aucun moment de solvant organique quelconque tel que notamment les acides carboxyliques C1 à C10, ni des alcools, éthers, cétones, esters, hydrocarbures (éventuellement chlorés) aliphatiques ou aromatiques, sulfoxydes, acétonitrile, amides etc.

Le procédé selon l'invention permet également de ne faire appel à aucun catalyseur d'ozonolyse, dont l'utilisation entrainerait des coûts supplémentaires et problèmes de gestion de déchets. Les catalyseurs homogènes connus pour l'ozonolyse comprennent notamment des acides de Lewis, par exemple le BF₃, et les acides de Bronsted tel que les acides minéraux (par exemple, l'acide sulfurique). Des catalyseurs hétérogènes solides sont également connus dans ce contexte tel que des résines acides, résines échangeuses d'ions, zéolites, catalyseurs à base de métaux de transition (Fe, Mn, Ni, Co, Cu ...) notamment sous forme d'oxydes et/ou déposés sur des oxydes supports tels que l'alumine, la silice ou l'oxyde de titane.

Le procédé selon l'invention de transformation d'acide oléique en acides azélaïque et pélargonique est mené jusqu'à l'obtention des produits finals (acides azélaïque et pélargonique) sans rajout à aucun moment de catalyseur comme notamment détaillé ci-dessus ni de base forte ni d'acide fort (dont certains peuvent être employé en tant que catalyseur). Par « acide fort » on entend des acides de Bronsted contenant un atome d'hydrogène se séparant (quasi-)totalement dans l'eau (dissociation (quasi-) totale, pKₐ < 0), par exemple les acides chlorhydrique (HCl), sulfurique (H₂SO₄) et nitrique (HNO₃). Par « base forte » on entend des bases ayant une valeur pKₐ > 14 - il s'agit souvent dans un contexte industriel d'oxydes et d'hydroxydes de métaux et notamment de métaux alcalins et de métaux alcalino-terreux, par exemple la soude (NaOH) et la potasse (KOH).

Une séquence avantageuse de la nouvelle synthèse simultanée des acides azélaïque et pélargonique par l'ozone à partir d'eau dispersée dans l'acide oléique ainsi que les étapes opératoires avantageuses d'un mode de réalisation du nouveau procédé sont données ci-après à titre d'exemple non limitatif.
Première étape : l'eau sous forme liquide à température ambiante (20°C ± 5°C) est avantageusement dispersée dans l'acide oléique de qualité technique (pureté minimale de 80% m/m) par une agitation mécanique soutenue jusqu'à obtention d'une dispersion homogène.
Deuxième étape : la dispersion homogène ainsi constituée est avantageusement soumise à un flux d'ozone constitué de fines bulles animées d'une vitesse ascensionnelle verticale. La concentration de l'ozone dans son gaz vecteur peut être préférentiellement comprise entre 140 g d'O₃/m³ TPN et 220 g d'O₃/m³ TPN, plus préférentiellement entre 160 g d'O₃/m³ TPN et 200 g d'O₃/m³ TPN. Durant l'introduction d'ozone dans le réacteur, l'agitation mécanique est maintenue. Le réacteur dans lequel se déroule la réaction est équipé d'un dispositif à double enveloppe (« water jacket ») permettant le refroidissement du milieu réactionnel et son maintien à la température de 80°C maximum.
   Le réacteur est également équipé d'un dispositif de condensation des buées, lequel dispositif sera décrit ultérieurement.
Troisième étape : l'introduction du flux d'ozone est maintenue jusqu'à ce que l'absorption de l'ozone par le milieu réactionnel soit quasi nulle (contrôle par analyseur d'ozone résiduel).
Quatrième étape : le mélange réactionnel constitué d'acide azélaïque, d'acide pélargonique et d'eau, est chauffé jusqu'à une température de 85°C, puis transféré dans une cuve de cristallisation.
Cinquième étape : le volume de soluté est ajusté à la température 85°C de telle sorte que l'on garantisse l'absence totale de cristaux d'acide azélaïque dans la phase liquide.
Sixième étape : le soluté présent dans la cuve de cristallisation, est refroidi progressivement de telle sorte que l'on atteigne la température de 5°C. Durant cette opération d'abaissement de la température, on maintient une agitation du soluté.
Septième étape : les cristaux d'acide azélaïque formés durant l'abaissement de la température du soluté sont séparés de ce dernier par filtration ou centrifugation, puis lavés par de l'eau glacée.
Huitième étape : la masse cristalline d'acide azélaïque recueillie après filtration ou centrifugation est séchée par étuvage sous vide à une température ne dépassant pas 60°C. Le surnageant (soluté mère), mélange d'acide pélargonique et d'eau est extrait ; l'acide pélargonique étant récupéré par distillation simple.
Neuvième étape : si cela est nécessaire, l'acide azélaïque cristallisé ainsi obtenu peut être encore purifié par une dissolution à l'eau chaude à 85°C suivie par une recristallisation.

Durant l'ensemble des opérations d'ozonation, c'est-à-dire durant la phase où l'ozone est introduit dans le soluté pour obtenir l'acide azélaïque et simultanément l'acide pélargonique, l'ozone résiduel éventuellement présent en cours de réaction est avantageusement évacué vers une destruction thermique de l'ozone résiduel où ce dernier est transformé en dioxygène (O₂) avant rejet à l'atmosphère.

### Brève description des figures

Les Figures 1a, 1b et 1c représentent l'acide oléique, l'acide azélaïque et l'acide pélargonique respectivement.
La Figure 2 représente de façon schématique un réacteur d'ozonation avec son agitation et le matériel environnant.

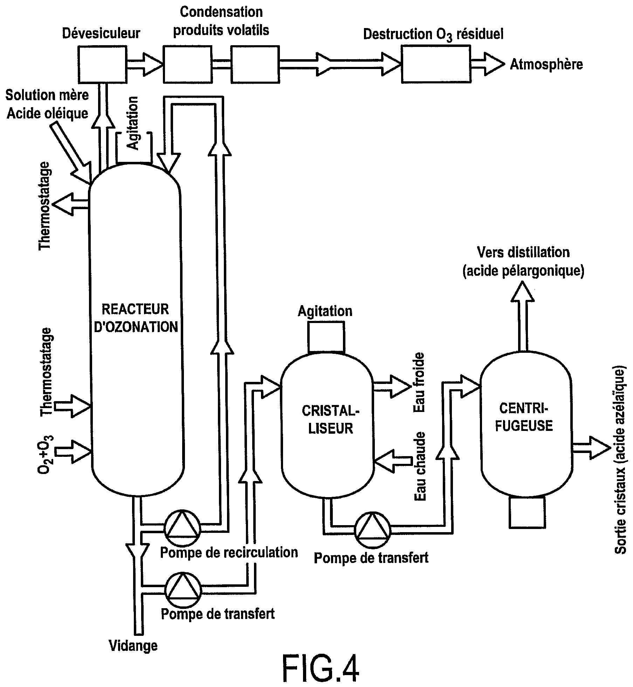

Les Figures 3 et 4 représentent de façon schématique des manières avantageuses de mettre en œuvre le procédé selon la présente invention.

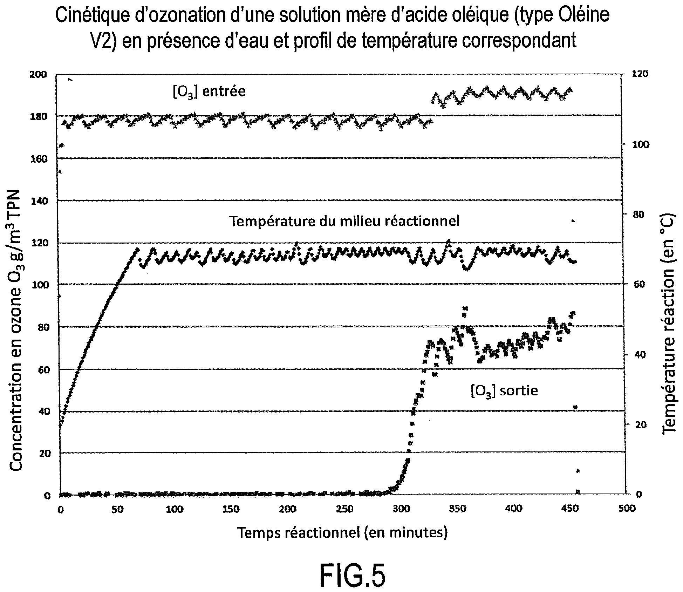

La Figure 5 montre la cinétique réactionnelle complète des deux phases constitutives du procédé de synthèse selon l'invention.

### Description détaillée de l'invention

La présente invention concerne notamment l'utilisation de l'ozone et un procédé de mise en œuvre de ce gaz réactif dans le but d'assurer la synthèse de l'acide azélaïque cristallisé à partir d'une solution mère liquide d'acide oléique de préférence d'origine uniquement végétale (origine biosourcée exclusive). La solution mère liquide d'acide oléique proviendra de façon avantageuse de graines de tournesol.

Une solution mère liquide d'acide oléique couramment disponible, par exemple contenant 80% m/m en acide oléique, 11% m/m en acide linoléique, et 9% m/m d'acides gras saturés (acide palimitique, acide stéarique...), peut être utilisée dans la nouvelle méthode de synthèse simultanée des acides azélaïque et pélargonique sans faire l'objet d'une purification préalable quelconque avant sa mise en œuvre dans le procédé suivant l'invention.

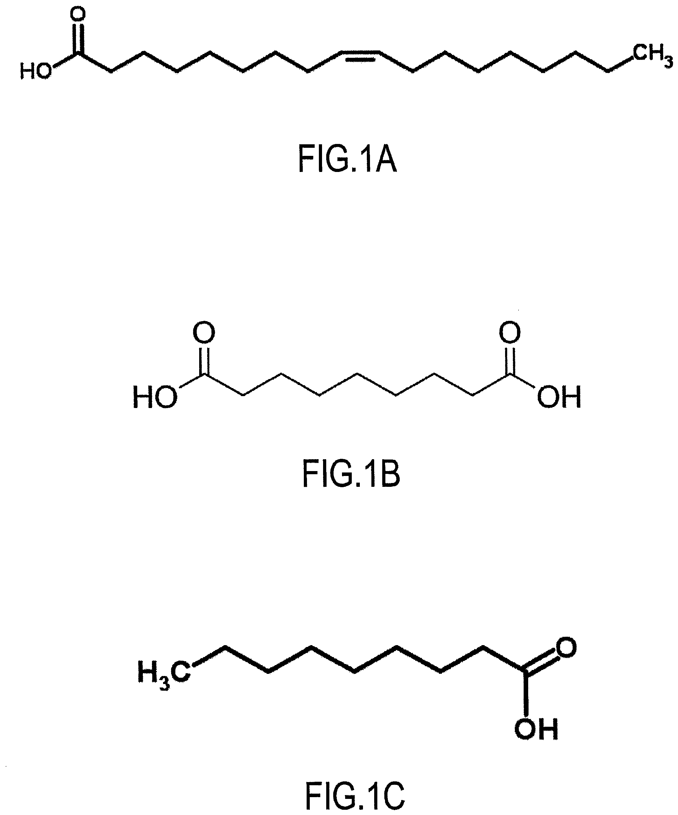

Dans le cadre de la présente invention, les acides azélaïque et pélargonique (Fig. 1b et 1c) peuvent être synthétisés à partir de l'acide oléique (Fig. 1a) mis en dispersion avec de l'eau ; dans cette dispersion l'ozone gazeux étant introduit avec son gaz vecteur (O₂) sous forme de fines bulles générées par un ou des diffuseurs poreux et cisaillées par l'action de turbines de type Rushton avec turbines intermédiaires d'accélération. Les équipages de turbines Rushton et de turbines intermédiaires d'accélération permettent l'homogénéité de la dispersion d'eau dans l'acide oléique puis, au cours de la réaction, l'homogénéité de la dispersion d'acide azélaïque, d'acide pélargonique et d'eau ainsi que l'accroissement de la surface spécifique d'échange entre le gaz (ozone + gaz vecteur) et la dispersion (acide azélaïque, acide pélargonique et eau) par cisaillement des bulles crées par les diffuseurs poreux. Au cours de la réaction la composition de la dispersion se modifie au fil du temps ; les acides azélaïque et pélargonique voient leurs quantités augmenter (croissance en fonction du temps) alors que la quantité d'acide oléique décroît en fonction du temps. La quantité d'eau, quant à elle, avantageusement maintenue essentiellement constante durant toute la réaction (variant de préférence de moins de 5% par rapport à sa quantité initiale, plus préférentiellement de moins de 1%).

De manière avantageuse, l'ozone utilisé pour la synthèse des acides azélaïque et pélargonique est fabriqué à partir d'oxygène liquide pur évaporé et de l'action de l'électricité utilisée sous forme de décharge de type couronne (Corona) ; l'ozone est donc un produit pur qui n'altère pas la qualité de produit bio-sourcé des acides azélaïque et pélargonique.

La Demanderesse a mis en évidence qu'un traitement par l'ozone d'une suspension aqueuse d'acide oléique, dans des conditions qui seront précisées dans les paragraphes qui suivent, permettait, en une opération unitaire d'oxydation, suivie d'une opération de cristallisation, elle-même suivie d'une opération de filtration ou de centrifugation, suivie d'une opération de lavage à l'eau glacée et d'un séchage par étuvage sous vide, d'obtenir :
- D'une part, des cristaux d'acide azélaïque d'une grande pureté (98% m/m ou plus) avec un rendement de transformation massique important
   - un rendement de transformation peut être observé au-delà de 55% (m/m).
- D'autre part, une solution d'acide pélargonique avec un rendement de transformation massique comparable à celui de l'acide azélaïque (soit +55% m/m) dans la mesure où cette réaction répond aux mêmes constantes cinétiques que celles observées pour l'acide azélaïque (réaction équimolaire à partir de l'acide oléique).

Comparé aux bilans énergétiques des solutions techniques actuelles, le procédé de l'invention présente un bilan énergétique intéressant et très inférieur aux méthodes chimiques actuelles. La consommation moyenne totale d'énergie électrique peut s'établir dans le cadre de la présente invention entre 10,0 kWh.kg⁻¹ d'acides azélaïque et pélargonique et 12,0 kWh.kg⁻¹ d'acides azélaïque et pélargonique. Ceci s'explique de par le fait que le seul réactif mis en œuvre est l'ozone et de par le fait qu'il n'y a aucune nécessité de traiter des dégagements gazeux ou des effluents liquides fortement contaminés.

L'ozone utilisé pour cette synthèse chimique est avantageusement produit *in situ* à partir d'oxygène liquide de grande pureté, présentant un point de rosée de l'ordre de -86°C. Après évaporation de cet oxygène liquide et détente jusqu'à la pression requise (2,5 Bar), l'oxygène gazeux extrêmement sec est admis dans le générateur d'ozone à la sortie duquel on obtient le gaz vecteur « oxygène », supportant la quantité d'ozone choisie à la concentration choisie.

La Demanderesse a mis en évidence que la synthèse des acides azélaïque et pélargonique à partir de l'acide oléique utilisant le réactif gazeux ozone, s'effectuait au mieux si une forte concentration en ozone est utilisée dans le gaz vecteur « oxygène ». De la même façon, la Demanderesse a mis en évidence que cette synthèse s'effectue de préférence en une seule phase.

Lors de la phase réactionnelle la concentration d'ozone utilisée est de préférence très élevée, de l'ordre de 160 à 200 g d'O₃/m³ TPN, et la durée maximale de la réaction est de préférence de l'ordre de 480 minutes, plus préférentiellement de l'ordre de 180 minutes. Des durées plus longues, d'au maximum 550 voire 600 minutes sont cependant envisageables, mais elles entraînent une cinétique réactionnelle plus lente et une consommation globale d'énergie plus importante. Avantageusement, pendant toute la durée de la réaction l'agitation mécanique est maintenue et une recirculation par une boucle externe du procédé est mise en œuvre afin d'améliorer le potentiel d'échange entre la phase gazeuse et la dispersion. La dispersion est prélevée en partie basse du réacteur, acheminée par une pompe de recirculation et réinjectée en partie haute du réacteur. Le débit de recirculation de la dispersion est avantageusement compris entre 200 et 600% du volume réactionnel, préférentiellement entre 400 et 500% du volume réactionnel. Cette particularité conceptuelle a pour avantage d'accroitre la vitesse de descente de la dispersion et de diminuer la vitesse ascensionnelle des bulles tout en conservant un potentiel d'échange très important (augmentation de la vitesse de descente de la dispersion = augmentation de l'hydrodynamique à la paroi des bulles).

A l'issue de la phase proprement dite d'ozonation, la dispersion mère et les acides formés (acides azélaïque et pélargonique) sont avantageusement transvasés dans un réacteur à double enveloppe permettant de chauffer le mélange jusqu'à une température de 85°C ; ce chauffage s'effectuant sous légère agitation. Après stabilisation de la température au niveau requis, la dispersion mère et les acides formés peuvent être transvasés dans un réacteur de cristallisation.

Dans le réacteur de cristallisation, la température de la suspension (acides azélaïque et pélargonique + eau) est avantageusement abaissée progressivement jusqu'à atteindre 5°C sous légère agitation. Les cristaux d'acide azélaïque formés sont avantageusement séparés de la solution mère par filtration ou centrifugation puis, lavés par de l'eau glacée (T<5°C).

Selon un mode avantageux de la présente invention, l'acide azélaïque est obtenu sous forme de cristaux et lavé à l'eau pure (déionisée ou déminéralisée) ou de type eau potable.

La masse cristalline d'acide azélaïque est séchée par étuvage sous vide à une température qui de préférence n'excédera pas 60°C. Les cristaux d'acide azélaïque obtenus présentent avantageusement, après séchage, une pureté supérieure à 98% avec une teneur résiduelle en humidité de 1% (m/m).

En parallèle, le surnageant (mélange d'acide pélargonique et d'une petite quantité d'eau) peut être conduit vers une colonne de distillation dans laquelle la séparation des deux phases sera opérée et l'acide pélargonique récupéré.

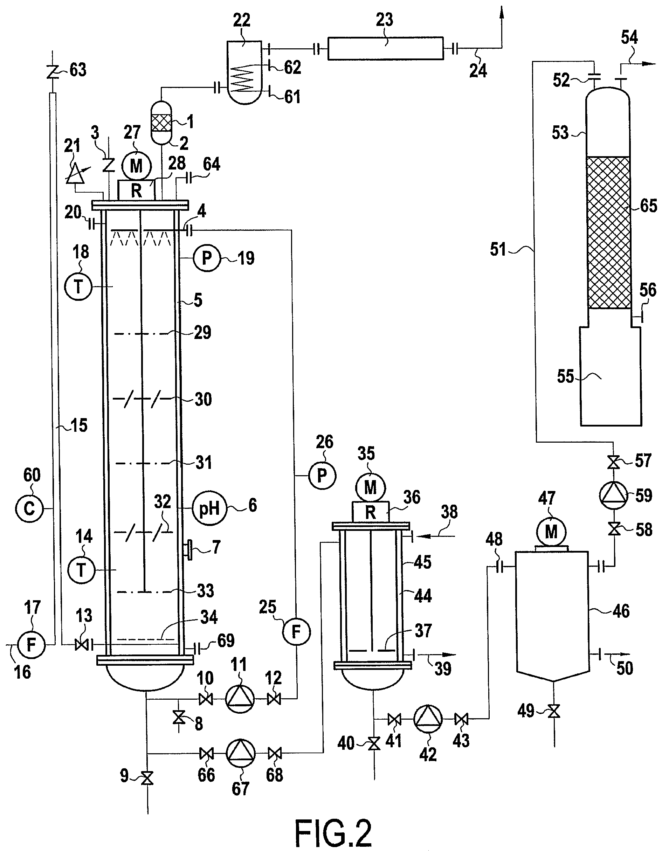

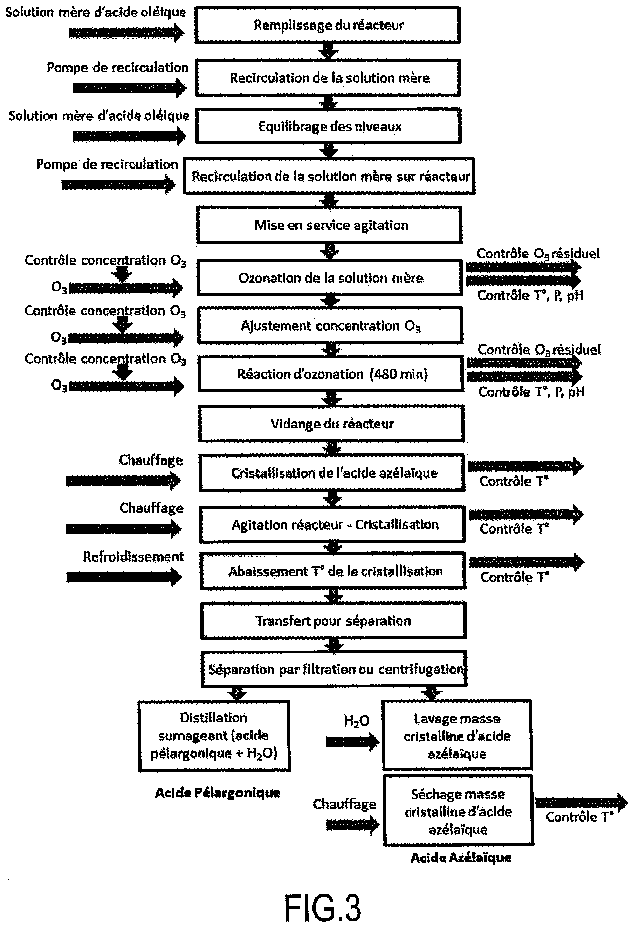

Dans un mode avantageux de mise en œuvre de l'invention, le traitement de la solution liquide mère d'acide oléique par l'ozone se fait suivant un procédé décrit par les Figures 3 (diagramme des opérations) et 4 (schéma bloc du procédé) et des moyens généraux et de détail du procédé décrits à la Figure 2. Les repères portés sur le schéma général de la Figure 2, qui représente un exemple non limitatif d'une installation et du procédé qui convient pour la mise en œuvre de la présente invention, désignent les éléments suivants :
- **1** dévésiculeur
- **2** orifice d'extraction de l'ozone résiduel
- **3** casse vide
- **4** retour de la recirculation de la solution mère d'acide oléique en boucle et dispositif d'introduction et de répartition de cette solution dans le réacteur
- **5** chemise d'eau (« water jacket »)
- **6** mesure de pH
- **7** hublot de visualisation de la réaction
- **8** prise d'échantillon
- **9** vidange du réacteur
- **10** vanne d'extraction de la solution mère d'acide oléique ozoné vers boucle de recirculation
- **11** pompe de recirculation
- **12** vanne de sectionnement de la boucle de recirculation
- **13** vanne de sectionnement de l'entrée d'oxygène ozoné
- **14** mesure de température du milieu réactionnel
- **15** lyre de dégardage de l'alimentation en oxygène ozoné
- **16** arrivée d'oxygène ozoné
- **17** mesure de débit d'oxygène ozoné
- **18** mesure de température
- **19** mesure de pression
- **20** sortie du fluide de refroidissement du « water jacket » (chemise d'eau)
- **21** soupape de sécurité
- **22** dispositif de condensation des volatils issus de la réaction
- **23** destruction de l'ozone résiduel en excès
- **24** mise à l'atmosphère
- **25** mesure de débit de recirculation d'acide oléique
- **26** mesure de pression de la boucle de recirculation de l'acide oléique
- **27** moteur d'entrainement des mobiles d'agitation
- **28** motoréducteur des mobiles d'agitation
- **29** mobile d'agitation
- **30** mobile d'accélération
- **31** mobile d'agitation
- **32** mobile d'accélération
- **33** mobile d'agitation
- **34** dispositif de diffusion d'ozone
- **35** moteur d'entrainement de l'agitation du cristalliseur
- **36** motoréducteur du mobile d'agitation du cristalliseur
- **37** mobile d'agitation
- **38** entrée du circuit du « water jacket »
- **39** sortie du circuit du « water jacket »
- **40** vidange du cristalliseur
- **41** vanne d'isolement du circuit de transfert d'acide azélaique et d'acide pélargonique
- **42** pompe de transfert des acides azélaique et pélargonique
- **43** vanne de sectionnement du circuit de transfert des acides azélaique et pélargonique
- **44** water jacket (chemise d'eau) du cristalliseur
- **45** cristalliseur
- **46** centrifugeuse
- **47** moteur d'entrainement de la centrifugeuse
- **48** entrée d'acides azélaique et pélargonique dans la centrifugeuse
- **49** vidange centrifugeuse
- **50** sortie des cristaux d'acide azélaique
- **51** circuit de sortie de l'acide pélargonique et de l'eau
- **52** entrée sur la colonne de distillation
- **53** colonne de distillation
- **54** sortie des vapeurs d'acide pélargonique
- **55** réservoir de pieds de la colonne de distillation
- **56** entrée de vapeur
- **57** vanne de sectionnement du circuit de transfert d'acide pélargonique et d'eau
- **58** vanne de sectionnement du circuit de transfert d'acide pélargonique et d'eau
- **59** pompe de transfert
- **60** contrôle de la concentration en ozone du gaz ozoné
- **61** entrée du circuit de refroidissement du condenseur
- **62** sortie du circuit de refroidissement du condenseur
- **63** dispositif casse vide de la lyre de dégardage
- **64** introduction de l'acide oléique dans le réacteur
- **65** garnissage de la colonne de distillation
- **66** vanne de sectionnement du circuit de transfert des acides azélaique et pélargonique
- **67** pompe de transfert des acides azélaique et pélargonique
- **68** vanne de sectionnement du circuit de transfert des acides azélaique et pélargonique
- **69** entrée du « water jacket » du réacteur d'ozonation

La synthèse des acides azélaique et pélargonique à partir de l'acide oléique commence de façon appropriée par le remplissage de l'installation aux niveaux requis. Le remplissage se fait à partir de l'entrée repérée (64).

De manière avantageuse, une fois le remplissage assuré au niveau requis et le débit de recirculation ajusté, la masse d'eau requise est mise en dispersion et la phase d'ozonation peut démarrer.

Compte tenu des concentrations en ozone dans le gaz vecteur préférables pour la synthèse des acides azélaique et pélargonique (160 à 200 g.m⁻³ TPN) le gaz vecteur préféré pour la génération de l'ozone est de l'oxygène pur et sec issu de l'évaporation d'oxygène liquide, afin de pouvoir d'atteindre les concentrations citées précédemment. L'oxygène pur et sec issu de l'évaporation d'oxygène liquide est donc à préférer à d'autres gaz vecteurs (air sec ou mélange air sec + O₂).

Le gaz vecteur oxygène supportant l'ozone à forte concentration peut être introduit dans le réacteur par l'intermédiaire de l'arrivée (16) et le débit contrôlé par le dispositif de mesure de débit (17). La concentration d'ozone dans le gaz vecteur peut être mesurée par l'analyseur (60). Avant son introduction dans le réacteur, le gaz ozoné parcourt de manière avantageuse la lyre de dégardage (15), l'introduction proprement dite dans le réacteur s'effectuant par la vanne de sectionnement (13) qui alimente le dispositif interne de diffusion (de bullage) (34).

Le dispositif de bullage est avantageusement constitué par des disques poreux montés sur support capables de créer des bulles de taille comprise entre 2 et 4 mm. Le gaz ozoné transformé en bulles traverse la masse de solution d'acide oléique en cours de réaction. Dès l'établissement du flux de gaz ozoné, dès que les bulles traversent la solution d'acide oléique, l'agitation est mise en service pour fragmenter les bulles de gaz ozoné et intéresser la totalité de la masse réactionnelle.

Le dispositif d'agitation est préférentiellement constitué par un équipage de mobiles, fixés sur un même arbre d'entrainement (29)(30)(31)(32)(33).

La Demanderesse a pu observer que la dispersion de fines bulles était optimale lorsque l'on utilisé un équipage multiple de mobiles constitué de la façon suivante :
- 3 mobiles d'agitation de type Rushton ou similaires,
- 2 mobiles d'accélération intercalés entre les mobiles d'agitation.

Cette disposition permet de rompre le spectre des lignes de vitesse générées par les turbines de Rushton.

Durant toute la durée de la réaction, une recirculation est avantageusement mise en action à partir d'un piquage installé en fonds de réacteur, celui-ci étant muni d'une prise d'échantillon (8). La recirculation de l'acide oléique est favorisée par l'utilisation d'une pompe (11) et de deux vannes de sectionnement (10) et (12). Le débit dans la boucle de circulation peut être contrôlé par un débimètre (25) et une mesure de pression (26). L'acide oléique en recirculation peut être introduit en partie haute du réacteur (4) par un dispositif permettant la distribution et l'aspersion de l'acide oléique en recirculation sur la masse d'acide oléique en réaction.

Cette recirculation de l'acide oléique en réaction permet d'augmenter l'hydrodynamique de l'acide oléique vis-à-vis des bulles de gaz ozoné et par ce biais de favoriser l'échange gaz-liquide en se rapprochant d'un système réputé piston.

La Demanderesse a constaté que le débit de recirculation de l'acide oléique en cours de réaction est avantageusement compris entre 200% et 600% de la masse en réaction, préférentiellement entre 400% et 500% de la masse d'acide oléique en réaction.

Durant toute la réaction, la masse réactionnelle est avantageusement refroidie par l'intermédiaire du « water jacket » (chemise d'eau) équipant le réacteur. L'eau glacée de refroidissement entre par l'orifice (69) et ressort par l'orifice (20).

Des mesures de température (14) (18) permettent le contrôle de la température du milieu réactionnel et l'ajustement éventuel du débit d'eau glacée alimentant le « water jacket » (chemise d'eau).

Une mesure de pression (19) permet le contrôle de la pression dans le ciel gazeux. Une mesure de pH (6) permet le contrôle du pH du milieu réactionnel.

Un hublot de visualisation (7) permet le contrôle de la dispersion des bulles de gaz et de l'effet des mobiles d'agitation.

Le réacteur est avantageusement mini d'une soupape de sécurité tarée (21), d'un casse vide (3). Après réaction le gaz ozoné résiduel s'échappe du réacteur par l'orifice (2) au travers d'un dévésiculeur (1). A la sortie du dévésiculeur, le gaz est avantageusement admis dans un dispositif de récupération des produits volatils (22) qui sont condensés par l'action de la température générée par un circuit d'eau glacée. Ce circuit d'eau glacée comporte ici une entrée (61) et une sortie (62). En sortie du dispositif de récupération des produits volatils, l'oxygène contenant l'ozone résiduel en excès est dirigé vers un dispositif de destruction de l'ozone en excès (23). A la sortie de ce destructeur d'ozone en excès l'oxygène, débarrassé des traces d'ozone, peut être rejeté dans l'atmosphère par la tuyauterie (24).

Une fois la réaction terminée, au bout de 180 minutes environ selon un mode de réalisation préférentiel, la totalité de l'acide oléique a été transformé en acide azélaique et pélargonique. Le réacteur est donc vidangé par l'intermédiaire de la pompe (67) et des vannes de sectionnement (67) et (68).

Le mélange des deux acides azélaique et pélargonique est avantageusement introduit dans un cristalliseur (45), où sa température est graduellement abaissée jusqu'à 5°C par l'intermédiaire du « water jacket » (44) équipant le cristalliseur ; lequel « water jacket » est alimenté par de l'eau glacée qui rentre par l'orifice (39) et ressort par l'orifice (38). Durant toute l'opération de refroidissement, le mélange des deux acides (acide azélaique et acide pélargonique) est avantageusement agité au moyen d'un mobile d'agitation (37), d'un moteur d'entrainement (35) et d'un réducteur (36). Lorsque la température de 5°C est atteinte et stabilisée, les cristaux d'acide azélaique et l'acide pélargonique sont transférés vers une centrifugeuse (46). Le transfert du cristalliseur vers la centrifugeuse se fait par l'intermédiaire de la pompe (42) et des vannes de sectionnement (41) et (43). Une vanne (40) permet la vidange du cristalliseur ainsi que l'évacuation des eaux de nettoyage. L'introduction des cristaux d'acide azélaique et d'acide pélargonique dans la centrifugeuse se fait avantageusement par l'intermédiaire de l'entrée (48). Le panier de la centrifugeuse est ici entrainé par le moteur (47). Le bac de centrifugation peut être vidangé et les eaux de lavage évacuées par la vanne (49).

Après centrifugation les cristaux d'acide azélaique sont avantageusement récupérés par l'orifice (50) cependant que le surnageant (acide pélargonique) est transféré par l'intermédiaire de la pompe (59) et des vannes (57) et (58) par l'intermédiaire du circuit (51) vers le distillateur (53). L'acide pélargonique entre avantageusement dans le distillateur par l'orifice (52), il traverse le corps de garnissage (65) parcouru à contre-courant par de la vapeur d'eau introduite par l'orifice (56). L'acide pélargonique débarrassé de ses fractions aqueuses et récupéré en (54) cependant que les eaux mères présentes sont condensées et recueillies dans le bac de pieds (55).

### Résultats obtenus

L'ensemble des conditions opératoires préférentielles identifiées et des résultats obtenus sont présentés dans le tableau I ci-après.

**Tableau I**

| **Synthèse des conditions opératoires et des résultats obtenus** | |
|---|---|
| **Rubriques** | **Valeurs** |
| Concentration en acide oléique de la solution mère (C_{M}) | C_{M} ≥ 800 g.L⁻¹ |
| Nombre d'opérations chimiques de la synthèse | 1 |
| Nombre de phases enchaînées de la synthèse | 0 |
| Nombre d'opérations enchaînées de la synthèse | 4 |
| Gaz vecteur utilisé / pureté | O₂ / 99% |
| Concentration d'ozone (C) | 160 < C < 200 g.m⁻³ TPN |
| Temps réactionnel (t_{R}) | 180 min |
| Température de départ de la réaction (Td) | Td = 20°C ± 5°C |
| Température de la réaction (T) | T ≤ 80°C |
| Rendement de la réaction chimique de transformation (acides azélaïque et pélargonique formés) (η) | η ≥ 75%(M/M) |
| Pureté de l'acide azélaïque cristallisé (P) | P ≥ 98% |
| Energie totale consommée pour la synthèse simultanée des acides azélaïque et pélargonique (E) | 10,0 KWh.kg⁻¹ < E< 12,0 KWh.kg⁻¹ |

Le procédé mis au point par la Demanderesse ne présente aucun dégagement gazeux et ne nécessite pas le traitement de gaz nocifs avant rejet à l'atmosphère. L'énergie totale consommée par la synthèse pour aboutir à la production simultanée des acides azélaïque et pélargonique, n'est que de 10,0 à 12,0 KWh.kg⁻¹. Enfin, cette nouvelle voie de synthèse simultanée des acides azélaïque et pélargonique est une voie propre et sobre, respectueuse de l'environnement et valorisant des matières premières totalement biosourcées.

## Revendications

1. Procédé de préparation simultanée des acides azélaïque et pélargonique comprenant les étapes successives suivantes :
(a) fourniture d'une solution mère liquide d'acide oléique ;
(b) dispersion d'une quantité d'eau dans la solution mère liquide d'acide oléique fournie à l'étape (a) sans employer d'acide carboxylique en tant que solvant organique, étant précisé que le pourcentage massique d'eau par rapport à la masse de solution mère liquide d'acide oléique lors de la réalisation de la dispersion est d'au moins 15% et d'au plus 34% ;
(c) mise en contact de la dispersion d'eau dans la solution mère liquide d'acide oléique, obtenue à l'étape (b), avec de l'ozone gazeux,
dans lequel on n'utilise aucun solvant organique, le procédé de transformation d'acide oléique en acides azélaïque et pélargonique étant mené jusqu'à l'obtention des acides azélaïque et pélargonique en utilisant l'eau comme unique solvant rajouté au mélange réactionnel, et
dans lequel le procédé est mené jusqu'à l'obtention des acides azélaïque et pélargonique sans que l'on ne mette en œuvre aucun catalyseur homogène ou hétérogène ni aucun acide fort ni aucune base forte.

2. Procédé selon la revendication 1, dans lequel la quantité en masse en eau dans le mélange réactionnel est maintenue essentiellement constante durant la totalité de la réaction, de façon à varier de moins de 5% par rapport à sa quantité initiale.

3. Procédé selon l'une quelconque des revendications 1 à 2, dans lequel la concentration de l'ozone gazeux à l'étape (c) est comprise entre 140 et 220 g.m⁻³ TPN, préférentiellement entre 160 et 200 g.m⁻³ TPN.

4. Procédé selon l'une quelconque des revendications 1 à 3, dans lequel la dispersion est réalisée à l'étape (b) et/ou maintenue pendant l'étape (c) par application d'un coefficient de cisaillement important **caractérisé par** une vitesse de rotation minimale des mobiles d'agitation d'au moins 170 rpm et d'au plus 270 rpm et/ou une valeur ASN (Apport Spécifique Nominal) d'au moins 30 et d'au plus 80 Watts/litre de dispersion.

5. Procédé selon la revendication 4, dans lequel la valeur ASN (Apport Spécifique Nominal) d'au moins 40 et d'au plus 60 Watts/litre de dispersion.

6. Procédé selon l'une quelconque des revendications 1 à 5, dans lequel la solution mère liquide d'acide oléique fournie à l'étape (a) comprend au moins 80% en masse d'acide oléique.

7. Procédé selon l'une quelconque des revendications 1 à 6, dans lequel la durée de l'étape (c), la mise en contact avec l'ozone gazeux est d'au plus 600 minutes, préférentiellement d'au plus 550 minutes.

8. Procédé selon la revendication 7, dans lequel la mise en contact avec l'ozone gazeux est d'au plus 180 minutes.

9. Procédé selon l'une quelconque des revendications 1 à 8, dans lequel l'ozone est généré sur le gaz vecteur oxygène pur.

10. Procédé selon l'une quelconque des revendications 1 à 9, comprenant les étapes supplémentaires suivantes :
(d) refroidissement de la dispersion d'acides azélaïque et pélargonique formée à l'étape (c) ;
(e) séparation par filtration ou centrifugation des cristaux d'acide azélaique formés à l'étape (c) et refroidie à l'étape (d) ;
(f) lavage à l'eau glacée des cristaux d'acide azélaique séparés à l'étape (e) ;
(g) distillation du mélange d'acide pélargonique et d'eau obtenu en tant que surnageant à l'étape (e).

11. Procédé selon l'une quelconque des revendications 1 à 10, dans lequel l'acide azélaïque est obtenu sous forme de cristaux et lavé à l'eau pure déionisée ou déminéralisée ou de type eau potable.

12. Procédé selon l'une quelconque des revendications 10 ou 11 dans lequel les cristaux d'acide azélaïque lavés sont ensuite soumis à un séchage.

## Patentansprüche

1. Verfahren zur gleichzeitigen Herstellung von Azelain- und Pelargonsäure, das die folgenden aufeinanderfolgenden Schritte umfasst:
a) Bereitstellen einer flüssigen Ölsäure-Stammlösung,
b) Dispersion einer Wassermenge in der in Schritt (a) bereitgestellten flüssigen Ölsäure-Stammlösung ohne Verwendung von Carbonsäure als organisches Lösungsmittel, wobei der Masseprozentanteil von Wasser, bezogen auf die Masse der flüssigen Ölsäure-Stammlösung, bei der Herstellung der Dispersion mindestens 15 % und höchstens 34 % beträgt,
(c) inkontaktbringen der in Schritt (b) erhaltenen Dispersion von Wasser in der flüssigen Ölsäure-Stammlösung mit gasförmigem Ozon,
wobei kein organisches Lösungsmittel verwendet wird und das Verfahren zur Umwandlung der Ölsäure in Azelain- und Pelargonsäure bis zum Erhalt der Azelain- und Pelargonsäure unter Verwendung von Wasser als einzigem dem Reaktionsgemisch zugegebenen Lösungsmittel durchgeführt wird, und
wobei das Verfahren bis zum Erhalt der Azelain- und Pelargonsäure durchgeführt wird, ohne dass dabei ein homogener oder heterogener Katalysator, eine starke Säure oder eine starke Base eingesetzt wird.

2. Verfahren nach Anspruch 1, bei dem die Wassermenge in dem Reaktionsgemisch während der gesamten Reaktion nahezu konstant gehalten wird, so dass sie in Bezug auf ihre ursprüngliche Menge um weniger als 5 % abweicht.

3. Verfahren nach einem der Ansprüche 1 bis 2, bei dem die Konzentration des gasförmigen Ozons in Schritt (c) zwischen 140 und 220 g.m⁻³ TPN, vorzugsweise zwischen 160 und 200 g.m⁻³ TPN liegt.

4. Verfahren nach einem der Ansprüche 1 bis 3, bei dem die Dispersion in Schritt (b) hergestellt und/oder während Schritt (c) aufrechterhalten wird, indem eine hohe Schergeschwindigkeit angewendet wird, **gekennzeichnet durch** eine minimale Drehzahl der Rührwerke von mindestens 170 U/min und höchstens 270 U/min und/oder einen ASN-Wert (apport specifique nominal) bzw. einen spezifischen Nominal-Energieeintrag von mindestens 30 und höchstens 80 Watt/Liter Dispersion.

5. Verfahren nach Anspruch 4, bei dem der ASN-Wert bzw. der spezifische Nominal-Energieeintrag mindestens 40 und höchstens 60 Watt/Liter Dispersion beträgt.

6. Verfahren nach einem der Ansprüche 1 bis 5, bei dem die in Schritt (a) bereitgestellte flüssige Ölsäure-Stammlösung mindestens 80 Masse-% Ölsäure umfasst.

7. Verfahren nach einem der Ansprüche 1 bis 6, bei dem die Dauer von Schritt (c), das Inkontaktbringen mit dem gasförmigen Ozon, höchstens 600 Minuten, vorzugsweise höchstens 550 Minuten beträgt.

8. Verfahren nach Anspruch 7, bei dem das Inkontaktbringen mit dem gasförmigen Ozon höchstens 180 Minuten beträgt.

9. Verfahren nach einem der Ansprüche 1 bis 8, bei dem das Ozon aus dem Trägergas reiner Sauerstoff erzeugt wird.

10. Verfahren nach einem der Ansprüche 1 bis 9, das die folgenden zusätzlichen Schritte umfasst:
(d) Abkühlen der in Schritt (c) gebildeten Dispersion von Azelain- und Pelargonsäure,
(e) Abscheiden der in Schritt (c) gebildeten und in Schritt (d) abgekühlten Azelainsäurekristalle durch Filtration oder Zentrifugation,
(f) Waschen der in Schritt (e) abgeschiedenen Azelainsäurekristalle mit Eiswasser,
(g) Destillieren des Gemisches aus Pelargonsäure und Wasser, das als Überstand in Schritt (e) erhalten wurde.

11. Verfahren nach einem der Ansprüche 1 bis 10, bei dem Azelainsäure in Kristallform erhalten und mit reinem entionisiertem oder demineralisiertem Wasser oder mit Trinkwasser gewaschen wird.

12. Verfahren nach einem der Ansprüche 10 oder 11, bei dem die gewaschenen Azelainsäurekristalle anschließend einer Trocknung unterzogen werden.

## Claims

1. Process for the simultaneous preparation of azelaic and pelargonic acids, comprising the following successive steps:
(a) providing a liquid stock solution of oleic acid;
(b) dispersing an amount of water in the liquid stock solution of oleic acid provided in step (a) without using carboxylic acid as organic solvent, it being specified that the mass percentage of water based on the mass of liquid stock solution of oleic acid when the dispersion is carried out is at least 15% and at most 34%;
(c) bringing the dispersion of water in the liquid stock solution of oleic acid, obtained in step (b), into contact with ozone gas,
wherein no organic solvents are used, the process of transforming oleic acid into azelaic and pelargonic acids being carried out until azelaic and pelargonic acids are obtained using water as the only solvent added to the reaction mixture, and
wherein the process is carried out until azelaic and pelargonic acids are obtained without using any homogeneous or heterogeneous catalyst or any strong acid or any strong base.

2. Process according to claim 1, wherein the mass amount of water in the reaction mixture is kept essentially constant throughout the entire reaction, so as to vary by less than 5% from its initial amount.

3. Process according to any one of claims 1 to 2, wherein the concentration of ozone gas in step (c) is between 140 and 220 g.m⁻³ NTP, preferentially between 160 and 200 g.m⁻³ NTP.

4. Process according to any one of claims 1 to 3, wherein the dispersion is carried out in step (b) and/or maintained during step (c) by applying a high shear coefficient **characterised by** a minimum rotation speed of the movable agitation means of at least 170 rpm and at most 270 rpm and/or an NSE (Nominal Specific Efficiency) value of at least 30 and at most 80 watts/litre of dispersion.

5. Process according to claim 4, wherein the NSE (Nominal Specific Efficiency) value of at least 40 and at most 60 watts/litre of dispersion.

6. Process according to any one of claims 1 to 5, wherein the liquid stock solution of oleic acid provided in step (a) comprises at least 80% by mass of oleic acid.

7. Process according to any one of claims 1 to 6, wherein the duration of step (c), the bringing into contact with ozone gas, is at most 600 minutes, preferentially at most 550 minutes.

8. Process according to claim 7, wherein the bringing into contact with ozone gas is at most 180 minutes.

9. Process according to any one of claims 1 to 8, wherein ozone is generated on the pure oxygen carrier gas.

10. Process according to any one of claims 1 to 9, comprising the following additional steps:
(d) cooling the dispersion of azelaic and pelargonic acids formed in step (c);
(e) separating by filtration or centrifugation the azelaic acid crystals formed in step (c) and cooled in step (d);
(f) washing with ice water the azelaic acid crystals separated in step (e);
(g) distilling the mixture of pelargonic acid and water obtained as supernatant in step (e).

11. Process according to any one of claims 1 to 10, wherein azelaic acid is obtained in the form of crystals and washed with pure deionised or demineralised water or drinking water.

12. Process according to any one of claims 10 or 11, wherein the washed azelaic acid crystals are then subjected to drying.
